# EUROPÄISCHE PATENTSCHRIFT

(11) **EP 3 029 048 B1**
(45) Veröffentlichungstag und Bekanntmachung des Hinweises auf die Patenterteilung: **12.04.2017**
(21) Anmeldenummer: 14196187.0
(22) Anmeldetag: 04.12.2014
(51) Int. Cl.: B01J 31/18, C07F 9/6574, C07B 41/06, C07C 45/50, C07F 15/00

(54) **Bisphosphite die einen unsymmetrischen Biphenol-Flügel-Baustein aufweisen**
Bis-phosphites with an asymmetric biphenol wing component
Bisphosphites présentant un composant périphérique en bisphénol asymétrique

(43) Veröffentlichungstag der Anmeldung: 08.06.2016
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Fridag, Dirk, 45721 Haltern am See (DE); Börner, Armin, 18059 Rostock (DE); Selent, Detlef, 18059 Rostock (DE)

(56) Entgegenhaltungen:
- WO-A1-2008/071508
- WO-A1-2014/056733
- ROBERT FRANKE ET AL: "Applied Hydroformylation", CHEMICAL REVIEWS, Bd. 112, Nr. 11, 14. November 2012 (2012-11-14), Seiten 5675-5732, XP055091930, ISSN: 0009-2665, DOI: 10.1021/cr3001803

## Beschreibung

Die Erfindung betrifft Bisphosphite, die mindestens einen unsymmetrischen Biphenol-Flügel-Baustein aufweisen. Des Weiteren deren Verwendung als Liganden in der Hydroformylierung.

Ein Bisphosphit weist einen Zentral-Baustein, das so genannte Rückgrat, und zwei Flügel-Bausteine auf, welche mit dem Zentral-Baustein über das P-Atom verbunden sind. Die beiden Flügel-Bausteine können hierbei gleich, oder auch verschieden sein.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor P^{III}. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Die Patente US 4 694 109 und US 4 879 416 beschreiben Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten erreicht. In WO 95/30680 werden zweizähnige Phosphinliganden und ihr Einsatz in der Katalyse, unter anderem auch in Hydroformylierungsreaktionen, offenbart.

In DE 10 2006 058 682 A1 werden Bisphosphite offenbart, welche unterschiedliche aber symmetrischen Flügel-Bausteine aufweisen, wie beispielsweis die Verbindung Ib auf Seite 8 der DE 10 2006 058 682 A1.

WO2014/056733 offenbart asymmetrische Biphosphit-Liganden, deren Asymmetrie durch zwei unterschiedlich substituierte [1,3,2]-Dioxaphosphepin-Einheiten bedingt ist. Weiterhin offenbart WO2014/056733 Metall-Komplexe mit solchen Liganden sowie deren Verwendung zur Hydroformylierung. WO2008/071508 offenbart asymmetrische Biphosphit-Liganden mit 2,2'-Bisphenol-Kerngerüst, deren Asymmetrie durch zwei unterschiedliche Cyclophosphite hervorgerufen wird.
Diese Dokument offenbart weiterhin Metall-Komplexe mit solchen Liganden und die Verwendung zur Hydroformylierung.

Obgleich eine Vielzahl von Liganden und ihre Verwendung in der Rhodium-katalysierten Hydroformylierung bekannt sind, ist es wünschenswert, neue Liganden mit verbesserten Eigenschaften zu entwickeln.

Der Erfindung lag die Aufgabe zugrunde, Bisphosphite bereitzustellen, welche gegenüber den bekannten Bisphosphiten vorteilhafte Eigenschaften in der Hydroformylierung aufweisen. Insbesondere bestand die Aufgabe darin, neue Liganden bereitzustellen, deren Einsatz gegenüber strukturverwandten Bisphosphiten, welche ebenfalls drei Biphenoleinheiten aufweisen, zu einer verbesserten Ausbeute führen. Die verbesserte Ausbeute sollte bei zumindest einem Olefin realisiert werden.

Die Aufgabe wird gelöst durch eine Verbindung nach Anspruch 1.

Verbindung, welche die allgemeine Struktur **I** aufweist: 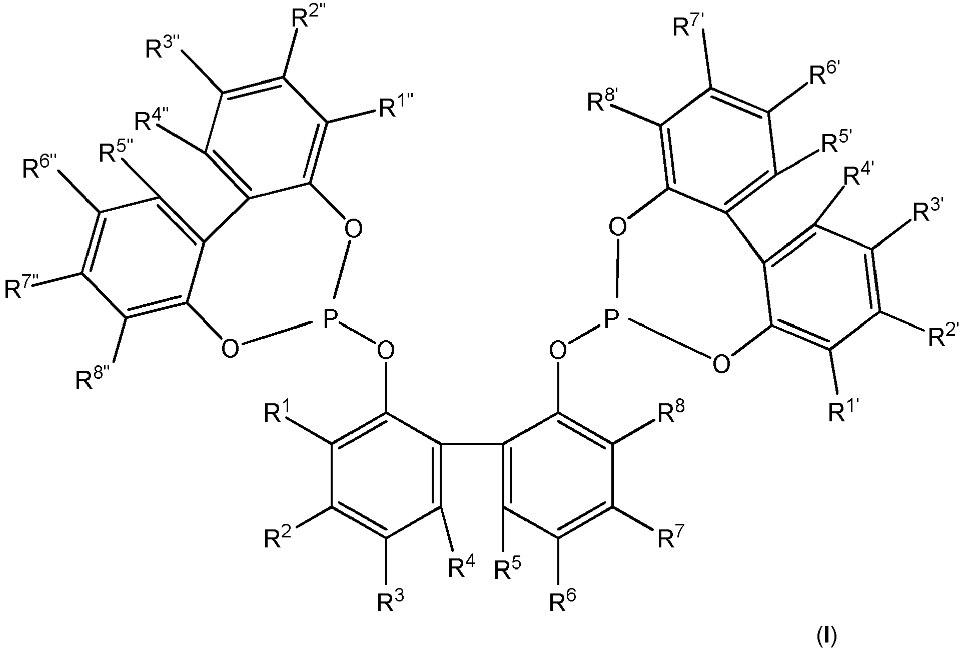 wobei
R¹, R², R³, R⁴, R⁵, R⁶, R⁷, R⁸ ausgewählt sind aus:
   -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, , -CO-(C₁-C₁₂)-Alkyl, -CO-(C₆-C₂₀)-Aryl, -COOH, -OH, -SO₃H, -CN, -NH₂, -N[(C₁C₁₂)-Alkyl]₂;
   R¹', R²', R³', R⁴', R⁵', R⁶', R⁷', R⁸', R¹", R²", R³", R⁴", R⁵", R⁶", R⁷", R⁸" ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, -CO-(C₁-C₁₂)-Alkyl, -CO-(C₆-C₂₀)-Aryl, -COOH, -OH, - SO₃H, -NH₂, -N[(C₁-C₁₂)-Alkyl]₂;
   wobei die genannten Alkyl- und Arylgruppen substituiert sein können,
   und die beiden Reste mindestens eines der vier folgenden Restepaare nicht für den gleichen Rest stehen: R¹' und R⁸', R²' und R⁷', R³' und R⁶', R⁴', und R⁵',
   und / oder die beiden Reste mindestens eines der vier folgenden Restepaare nicht für den gleichen Rest stehen: R¹" und R⁸", R²" und R⁷", R³" und R⁶", R⁴" und R⁵".

Durch das Merkmal "und die beiden Reste mindestens eines der vier folgenden Restepaare nicht für den gleichen Rest stehen: R¹' und R⁸', R²' und R⁷', R³' und R⁶', R⁴' und R⁵' und / oder die beiden Reste mindestens eines der vier folgenden Restepaare nicht für den gleichen Rest stehen: R¹" und R⁸", R²" und R⁷", R³" und R⁶", R⁴" und R⁵" " wird zum Ausdruck gebracht, dass es sich bei mindestens einem der beiden Biphenol-Flügel-Baustein um ein unsymmetrisches Biphenol handelt. Es besteht auch die Möglichkeit, dass beide Biphenol-Flügel-Baustein gleichzeitig unsymmetrisch sind. Bei unsymmetrischen Biphenolen lassen sich die beiden Aromaten nicht durch eine zwischen ihnen liegende Spiegelebene aufeinander abbilden.

Zugelassen sind folgende Restepaarungen, wie beispielsweise:
R¹' ungleich R⁸', R²' gleich R⁷', R³' gleich R⁶', R⁴' gleich R⁵',
R¹' gleich R⁸', R²' gleich R⁷', R³' ungleich R⁶', R⁴' gleich R⁵'.

Ober aber auch Restepaarungen bei denen mehr als nur ein Paar ungleich ist, wie beispielsweise:
R¹' ungleich R⁸', R²' gleich R⁷', R³, ungleich R⁶', R⁴' gleich R⁵';
R¹' ungleich R⁸', R²' ungleich R⁷', R³' ungleich R⁶', R⁴' gleich R⁵'.

Ausgeschlossen wird lediglich der Fall, bei dem alle vier Restepaare jeweils paarweise für den gleichen Rest stehen:
R¹' gleich R⁸', R²' gleich R⁷', R³' gleich R⁶', R⁴' gleich R⁵'.
Hierbei würde es sich um ein symmetrisches Biphenol handeln.

Entsprechendes gilt analog für die Reste mit ".

(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.

(C₆-C₂₀)-Aryl und -(C₆-C₂₀)-Aryl-(C₆-C₂₀)-Aryl- können jeweils unsubstituiert oder durch einen oder mehrere gleiche oder verschiedene Reste substituiert sein, die ausgewählt sind unter -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.
Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -(C₃-C₁₂)-Heterocycloalkylgruppen Tetrahydrothiophenyl, Tetrhydrofuryl, Tetrahydropyranyl und Dioxanyl.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter - H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I),-COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂. Substituierte -(C₆-C₂₀)-Arylgruppen und -(C₆-C₂₀)-Aryl-(C₆-C₂₀)-Arylgruppen sind vorzugsweise substituierte -(C₆-C₁₀)-Arylgruppen und -(C₆-C₁₀)-Aryl-(C₆-C₁₀)-Arylgruppen, insbesondere substituiertes Phenyl oder substituiertes Naphthyl oder substituiertes Anthracenyl. Substituierte -(C₆-C₂₀)-Arylgruppen tragen vorzugsweise eine oder mehrere z.B. 1, 2, 3, 4 oder 5 Substituenten, ausgewählt unter -(C₁-C₁₂)-Alkylgruppen, -(C₁-C₁₂)-Alkoxygruppen.

In einer Ausführungsform sind R¹, R², R³, R⁴, R⁵, R⁶, R⁷, R⁸ ausgewählt aus:
-H, -(C₁-C₁₂)-Alkyl, -O-(C₁-C₁₂)-Alkyl, -O-(C₆-C₂₀)-Aryl, -S-Alkyl, -S-Aryl.

In einer Ausführungsform sind R¹', R²', R³', R⁴', R⁵', R⁶', R⁷', R⁸' ausgewählt aus:
-H, -(C₁-C₁₂)-Alkyl, -O-(C₁-C₁₂)-Alkyl, -O-(C₆-C₂₀)-Aryl, -S-Alkyl, -S-Aryl.

In einer Ausführungsform sind R¹", R²", R³", R⁴", R⁵", R⁶", R⁷", R⁸" ausgewählt aus:
-H, -(C₁-C₁₂)-Alkyl, -O-(C₁-C₁₂)-Alkyl, -O-(C₆-C₂₀)-Aryl, -S-Alkyl, -S-Aryl.

In einer Ausführungsform sind R¹, R², R³, R⁴, R⁵, R⁶, R⁷, R⁸ ausgewählt aus:
-H, -(C₁-C₁₂)-Alkyl, -O-(C₁-C₁₂)-Alkyl, -O-(C₆-C₂₀)-Aryl.

In einer Ausführungsform sind R¹', R²', R³', R⁴', R⁵', R⁶', R⁷', R⁸' ausgewählt aus:
-H, -(C₁-C₁₂)-Alkyl, -O-(C₁-C₁₂)-Alkyl, -O-(C₆-C₂₀)-Aryl.

In einer Ausführungsform sind R¹", R²", R³", R⁴", R⁵", R⁶", R⁷", R⁸" ausgewählt aus:
-H, -(C₁-C₁₂)-Alkyl, -O-(C₁-C₁₂)-Alkyl, -O-(C₆-C₂₀)-Aryl.

In einer Ausführungsform stehen R¹, R¹' und R¹" für den gleichen Rest und
stehen R⁸, R⁸' und R⁸" für den gleichen Rest.

In einer Ausführungsform stehen R¹' und R⁸' nicht für den gleichen Rest und
stehen R¹" und R⁸" nicht für den gleichen Rest.

In einer Ausführungsform stehen die beiden Reste mindestens eines der vier folgenden Restepaare nicht für den gleichen Rest: R¹' und R⁸', R²' und R⁷', R³' und R⁶', R⁴' und R⁵', und stehen die beiden Reste mindestens eines der vier folgenden Restepaare nicht für den gleichen Rest: R¹" und R⁸", R²" und R⁷", R³" und R⁶", R⁴" und R⁵".

In einer Ausführungsform stehen die beiden Reste mindestens eines der vier folgenden Restepaare nicht für den gleichen Rest: R¹' und R⁸', R²' und R⁷', R³' und R⁶', R⁴, und R⁵',
und stehen die beiden Reste der vier folgenden Restepaare für den gleichen Rest: R¹" und R⁸", R²" und R⁷", R³" und R⁶", R⁴" und R⁵".

In einer Ausführungsform unterscheidet sich mindestens einer der vier genannten Reste von den anderen Resten: R¹', R⁸', R¹", R⁸".

In einer Ausführungsform unterscheidet sich mindestens einer der vier genannten Reste von den anderen Resten: R²', R⁷', R²", R⁷".

In einer Ausführungsform unterscheidet sich mindestens einer der vier genannten Reste von den anderen Resten: R³', R⁶', R³", R⁶".

In einer Ausführungsform unterscheidet sich mindestens einer der vier genannten Reste von den anderen Resten: R⁴', R⁵', R⁴", R⁵".

In einer Ausführungsform stehen die drei Reste der folgenden Dreiergruppen jeweils für den gleichen Rest:
R¹ gleich R¹' gleich R¹" ,
R² gleich R²' gleich R²" ,
R³ gleich R³' gleich R³" ,
R⁴ gleich R⁴' gleich R⁴" ,
R⁵ gleich R⁵' gleich R⁵",
R⁶ gleich R⁶' gleich R⁶".

In einer Ausführungsform weist die Verbindung die Formeln (1) auf:

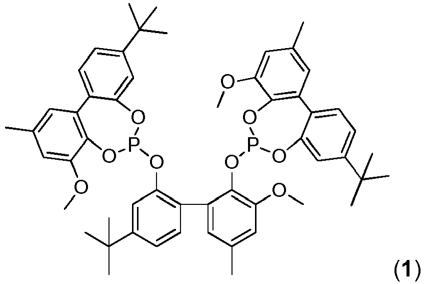

Neben den Verbindungen wird auch ein Komplex beansprucht, welcher diese Verbindungen umfasst.

Komplex umfassend:
- eine zuvor beschriebene Verbindung,
- ein Metallatom ausgewählt aus: Rh, Ru, Co, Ir.

In einer bevorzugten Ausführungsform ist das Metall Rh.

Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren wird die Verwendung der Verbindung als Ligand in einem Ligand-Metall-Komplex zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Das Verfahren bei dem die Verbindung als Ligand in einem Ligand-Metall-Komplex zur Umsetzung eines Olefins zu einem Aldehyd eingesetzt wird, wird ebenfalls beansprucht.

Verfahren umfassend die Verfahrensschritte:
a) Vorlegen eines Olefins,
b) Zugabe eines zuvor beschriebenen Komplexes,
   oder einer zuvor beschriebenen Verbindung und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

Es kann hierbei auch ein Überschuss an Liganden verwendet werden und nicht zwangläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 160 °C und ein Druck von 1 bar bis 300 bar. Besonders bevorzugt sind eine Temperatur von 100 °C bis 160 °C und ein Druck von 15 bar bis 250 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1- oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische.

Im Folgenden wird die Erfindung anhand von Ausführungsbeispielen und einer Figur näher erläutert.

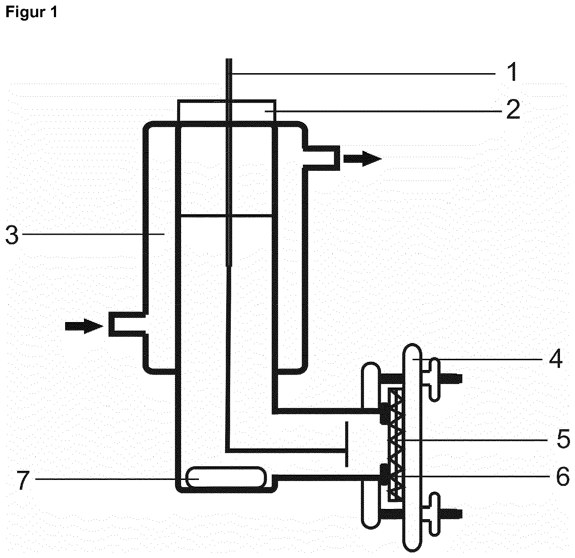

Die Figur 1 zeigt eine Reaktionsapparatur, in welcher die Kupplungsreaktion zu den entsprechenden unsymmetrischen Biarylen durchgeführt werden kann. Die Apparatur umfasst eine Nickelkathode (1) und eine Anode aus Bor-dotiertem Diamant (BDD) auf Silizium (5). Die Apparatur kann mit Hilfe des Kühlmantels (3) gekühlt werden. Die Pfeile deuten hierbei die Durchflussrichtung des Kühlwassers an. Der Reaktionsraum ist mit einem Teflonstopfen (2) verschlossen. Das Reaktionsgemisch wird durch ein Magnetrührstäbchen (7) durchmischt. Auf der anodischen Seite wird die Apparatur durch Schraubzwingen (4) und Dichtungen (6) verschlossen.

### Analytik

### Chromatographie

Die präparativen flüssigkeitschromatographischen Trennungen via "Flashchromatographie" wurden mit einem Maximaldruck von 1.6 bar an Kieselgel 60 M (0.040-0.063 mm) der Firma Macherey-Nagel GmbH & Co, Düren durchgeführt. Die Trennungen ohne Druckbeaufschlagung wurden an Kieselgel Geduran Si 60 (0.063-0.200 mm) der Firma Merck KGaA, Darmstadt durchgeführt. Die als Eluentien verwendeten Lösungsmittel (Essigsäureethylester (technisch), Cyclohexan (technisch)) wurden zuvor destillativ am Rotationsverdampfer gereinigt.
Zur Dünnschichtchromatographie (DC) wurden PSC-Fertigplatten Kieselgel 60 F254 der Firma Merck KGaA, Darmstadt verwendet. Die Rf-Werte sind in Abhängigkeit vom verwendeten Laufmittelgemisch angegeben. Zur Anfärbung der DC-Platten wurde eine Cer-Molybdatophosphorsäure-Lösung als Tauchreagenz verwendet. Cer-Molybdatophosphorsäure-Reagenz: 5.6 g Molybdatophosphorsäure, 2.2 g Cer(IV)-sulfat-Tetrahydrat und 13.3g konzentrierte Schwefelsäure auf 200 mL Wasser.

### Gaschromatographie (GC/GCMS)

Die gaschromatographischen Untersuchungen (GC) von Produktgemischen und Reinsubstanzen erfolgte mit Hilfe des Gaschromatographen GC-2010 der Firma Shimadzu, Japan. Es wird an einer Quarzkapillarsäule HP-5 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min) gemessen. Gaschromatographische Massenspektren (GCMS) von Produktgemischen und Reinsubstanzen wurden mit Hilfe des Gaschromatographen GC-2010 kombiniert mit dem Massendetektor GCMS-QP2010 der Firma Shimadzu, Japan aufgenommen. Es wird an einer Quarzkapillarsäule HP-1 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min; GCMS: Temperatur der Ionenquelle: 200 °C) gemessen.

### Schmelzpunkte

Schmelzpunkte wurden mit Hilfe des Schmelzpunktbestimmungsgerätes SG 2000 der Firma HW5, Mainz gemessen und sind unkorrigiert.

### Elementaranalyse

Die Elementaranalysen wurden in der analytischen Abteilung des Institutes für Organische Chemie der Johannes Gutenberg-Universität Mainz an einem Vario EL Cube der Firma Foss-Heraeus, Haunau angefertigt.

### Massenspektrometrie

Alle Elektrosprayionisation-Messungen (ESI+) wurden an einem QTof Ultima 3 der Firma Waters Micromasses, Milford, Massachusetts durchgeführt. EI-Massenspektren sowie die hochaufgelösten EI-Spektren wurden an einem Gerät des Typs MAT 95 XL Sektorfeldgerät der Firma ThermoFinnigan, Bremen, gemessen.

### NMR-Spektroskopie

Die NMR-spektroskopischen Untersuchungen wurden an Multikernresonanzspektrometern des Typs AC 300 oder AV II 400 der Firma Bruker, Analytische Messtechnik, Karlsruhe, durchgeführt. Als Lösungsmittel wurde CDCl3 verwendet. Die 1 H- und 13C-Spektren wurden gemäß dem Restgehalt an nicht deuteriertem Lösungsmittel nach der NMR Solvent Data Chart der Fa. Cambridge Isotopes Laboratories, USA, kalibriert. Die Zuordnung der 1 H- und 13C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

### Synthese der unsymmetrischen Biphenole

Die unsymmetrischen Biphenole wurden durch ein elektrochemisches Verfahren durch Kupplung von zwei Phenolen, welche sich in ihrem Oxidationspotential unterscheiden, hergestellt. Siehe hierzu auch B. Elsler, D. Schollmeyer, K. M. Dyballa, R. Franke, S. R. Waldvogel, "Metall- und reagensfreie hochselektive anodische Kreuzkupplung von Phenolen", Angew. Chem., 2014, DOI: 10.1002/ange.201400627

### Allgemeine Arbeitsvorschrift:

Die Kupplungsreaktion wurde in einer Apparatur durchgeführt, wie sie in Figur 1 dargestellt ist. 5 mmol des ersten Phenols mit einem Oxidationspotential *E_{Ox}1* werden mit 15 mmol des zweiten Phenols mit einem Oxidationspotential *E_{Ox}2* in den in der nachfolgenden Tabelle 1 angegebenen Mengen in 1,1,1,3,3,3-Hexafluorisopropanol (HFIP) und MeOH oder in Ameisensäure und MeOH gelöst. Die Elektrolyse erfolgt galvanostatisch. Der Außenmantel der Elektrolysezelle wird über einen Thermostaten auf etwa 10 °C temperiert, während die Reaktionsmischung gerührt und auf 50 °C mit Hilfe eines Sandbades erhitzt wird. Nach Ende der Elektrolyse wird der Zellinhalt mit Toluol in einen 50 mL Rundhalskolben überführt und das Lösungsmittel unter vermindertem Druck am Rotationsverdampfer bei 50 °C, 200-70 mbar entfernt. Nicht umgesetztes Edukt wird mittels Kurzwegdestillation zurückerhalten (100 °C, 10⁻³ mbar).

**Elektrodenmaterial**

| | |
|---|---|
| Anode: | Bor-dotierter Diamant (BDD) auf Si |
| Kathode: | Ni-Netz |

**Elektrolysebedingungen:**

| | |
|---|---|
| Temperatur [T]: | 50 °C |
| Stromstärke [I]: | 15 mA |
| Stromdichte [j]: | 2.8 mA/cm² |
| Ladungsmenge [Q]: | 2 F/mol Unterschusskomponente |
| Klemmspannung [Uₘₐₓ]: | 3-5 V |

Die Synthese der Biphenole erfolgte gemäß der oben beschriebenen allgemeinen Arbeitsvorschrift, und in einer Reaktionsapparatur, wie sie in Figur 1 dargestellt ist.

### 2,2'-Dihydroxy-3-methoxy-5-methyl-4'-(dimethylethyl)biphenyl

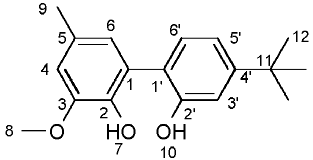

Es wurden 0.69 g (5 mmol, 1.0 Äquiv.) 4-Methylguajacol und 2.25 g (15 mmol, 3.0 Äquiv.) 3-tert-Butylphenol in 33 mL 1,1,1,3,3,3-Hexafluorisopropanol (HFIP) gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als farbloser Feststoff erhalten.
Ausbeute: 808 mg (63%, 3.1 mmol)
GC (Methode *hart*, HP-5): t_{R}= 13.97 min
R_{f}(CH:EE= 4:1)= 0.29
mₚ= 160.3 °C (aus DCM/CH umkristallisiert)
¹H-NMR (400 MHz, CDCl₃) δ= 1.37 (s, 9H, 12-H), 2.36 (s, 3H, 9-H), 3.94 (s, 3H, 8-H), 6.25 (s, 1 H, 7-H), 6.48 (s, 1 H, 10-H), 6.75 (d, 1 H, 6-H), 6.79 (d, 1 H, 4-H), 7.08 (dd, 1 H, 5'-H), 7.12 (d, 1H, 3'-H), 7.27 (d, 1 H, 6'-H);
Kopplungen: ⁴J_{4-H, 6}-_{H}= 1.7 Hz; ³J_{5'-H, 6'-H}= 8.0 Hz, ⁴J_{3'-H, 5'-H}= 1.7 Hz;
¹³C-NMR (101 MHz, CDCl₃) δ= 21.24 (C-9), 31.31 (C-12), 34.58 (C-11), 56.15 (C-8), 110.79 (C-4), 114.94 (C-3'), 118.30 (C-5'), 122.37 (C-1'), 123.88 (C-1), 123.94 (C-6), 130.45 (C-6'), 130.53 (C-4'), 139.24 (C-5), 146.32 (C-3), 152.91 (C-2'), 153.13 (C-2).
HRMS für C₁₅H₁₆O₄ (ESI+) [M+Na⁺]: ber: 309.1467, gef.: 309.1466
MS (EI, GCMS): m/z(%): 242 (100) [M]^{+·}, 227 (38) [M-CH3^{·}]⁺.
Elementaranalyse für C₁₈H₂₂O_{3:} ber: 75.50%, H: 7.74%, gef.: C: 75.41%, H: 7.72%.

### Synthese der Liganden

### 6,6'-((4'-((tert-Butyl)-3-methoxy-5-methyl-[1,1'-biphenyl]-2,2'-diyl)bis(oxy))bis(9-(tert-butyl)-4-methoxy-2-methyldibenzo[d,f][1,3,2]dioxaphosphepin).

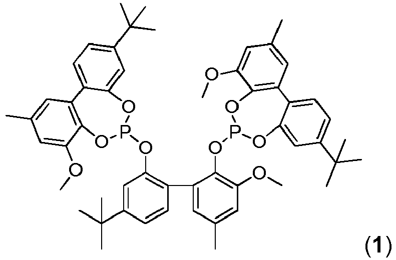

Eine Lösung von 4'-(*tert*-Butyl)-3-methoxy-5-methyl-[1,1'-biphenyl]-2,2'-diol (0,274 g; 0,957 mmol) in THF (10 ml) wurde mit einer Lösung von zwei Äquivalenten n-Butyllithium in Hexan (3,59 ml) bei -20 °C versetzt, die erhaltene Mischung noch 20 min bei dieser Temperatur gerührt und dann eine Lösung von 9-(*tert*-Butyl)-6-chloro-4-methoxy-2-methyldibenzo[*d*,*f*][1,3,2]dioxaphosphepin (0,792 g; 2,258 mmol) in THF (11 ml) bei Raumtemperatur zugegeben. Die Reaktionsmischung wurde über Nacht gerührt und das Lösungsmittel im Vakuum entfernt. Toluol (25 ml) wurde zugegeben und die resultierende Suspension filtriert. Das Filtrat wurde nochmals über Kieselgel filtriert und das Lösungsmittel im Vakuum entfernt. Der erhaltene Feststoff wurde für 3 h bei 50 °C / 0,1 mbar getrocknet. Ausbeute: 0,856 g (0,936 mmol; 98 %).
Elementaranalyse (ber. für C₅₄H₆₀O₉P₂ = 915,01 g/ mol) C 70,67 (70,88); H 6,52 (6,61); P 6,69 (6,77) %.
³¹P-NMR (CD₂Cl₂): 141,9 (d, *J*_{PP}= 7.8 Hz); 142,2 (d, *J*_{PP}= 7.8 Hz); 145,1 (d, *J*_{PP}= 7.8 Hz); 145,2 (d, *J*_{PP}= 7.8 Hz) ppm.
¹H-NMR (CD₂Cl₂): 1,22-1,33 (dd, 18 H); 1,37 (m, 9 H); 2,42 (m, 9 H); 3,81-3,88 (dd, 6 H); 4,02 (s, 3 H); 6,79-6,85 (m, 3 H, Hₐᵣₒₘ); 6,88 (m, 2 H, Hₐᵣₒₘ); 6,90-6,98 (m, 1 H, Hₐᵣₒₘ); 6,95 (m, 1 H, Hₐᵣₒₘ); 7,00-7,05 (m, 1 H, Hₐᵣₒₘ); 7,20-7,35 (m, 4 H, Hₐᵣₒₘ); 7,37-7,44 (m, 3 H, Hₐᵣₒₘ) ppm.

### 6,6'-((4'-(tert-Butyl)-3-methoxy-5-methyl-[1,1'-biphenyll-2,2'-diyl)bis(oxy))didibenzo[d,f][1,3,2]dioxaphosphepin.

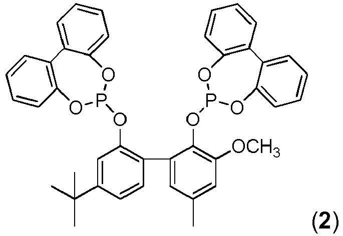

Eine Lösung von 4'-(*tert*-Butyl)-3-methoxy-5-methyl-[1,1'-biphenyl]-2,2'-diol (0,489 g; 1,708 mmol) in Toluol (12 ml) wurde mit Pyridin (0,389 g; 3,844 mmol) versetzt und die erhaltene Mischung bei 3 °C tropfenweise zu einer Lösung von 6-Chlorodibenzo[*d*,*f*][1,3,2]dioxaphosphepin (0,942 g; 3,758 mmol) in Toluol (12 ml) gegeben. Die Reaktionsmischung wurde über Nacht gerührt und dann filtriert. Das Filtrat wurde im Vakuum bis zur Trockne eingeengt und der erhaltene Feststoff bei 50 °C / 0,1 mbar getrocknet. Die zurückgebliebene Substanz wurde durch Säulenchromatografie gereinigt (Hexan/Toluol, 1:2, R*_{f}* = 0,3). Ausbeute: 0,738 g (1,032 mmol; 58 %).
Elementaranalyse (ber. für C₄₂H₃₆O₇P₂ = 714,69 g/ mol) C 70,59 (70,58); H 5,28 (5,08); P 8,85 (8,67) %.
³¹P-NMR (CD₂Cl₂): 144,3 (d, *J*_{PP} = 9,1 Hz); 148,1 (d, *J*_{PP} = 9,1 Hz) ppm.
¹H-NMR (CD₂Cl₂): 1,51 (m, 9 H); 2,45 (m, 3 H); 4,06 (s, 3 H); 6,80-6,87 (m, 3 H, Hₐᵣₒₘ); 6,98-7,03 (m, 2 H, Hₐᵣₒₘ); 7,03-7,05 (m, 1 H, Hₐᵣₒₘ); 7,28-7,35 (m, 8 H, Hₐᵣₒₘ); 7,35-7,38 (m, 1 H, Hₐᵣₒₘ); 7,38-7,43 (m, 2 H, Hₐᵣₒₘ); 7,46-7,54 (m, 4 H, Hₐᵣₒₘ) ppm.
¹³C-NMR (CD₂Cl₂): 21,5; 57,0; 113,8; 118,4 (d, *J*_{CP}= 10,1 Hz); 121,8; 122,5 (d, *J*_{CP}= 14,1 Hz); 124,9; 125,5 (d, *J*_{CP}= 17,3 Hz); 127,9; 128,7; 129,4; 129,4 (d, *J*_{CP}= 16,8 Hz); 130,1 (d, *J*_{CP}= 16,1 Hz); 131,3; 131,5; 131,6; 132,0; 134,4; 138,0; 149,5 (d, *J*_{CP}= 4,8 Hz); 149,7 (d, *J*_{CP}= 4,4 Hz); 149,8 (d, *J*_{CP}= 7,0 Hz); 151,2 (d, *J*_{CP}= 3,2 Hz); 153,4 ppm.

### Arbeitsvorschrift für die Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl getrocknet und unter Argon destilliert. Die als Substrat eingesetzten Substrate 1-Octen (Aldrich), cis/trans-2-Penten (Aldrich) und n-Octene (Oxeno GmbH, Octenisomerengemisch aus 1-Octen: ∼3 %; *cis*+*trans-*2-Octen; ∼49%; *cis*+*trans-*3-Octen: ∼29 %; *cis*+*trans-*Octen-4: -16 %; gerüstisomere Octene: -3 %) wurden mehrere Stunden über Natrium am Rückfluß erhitzt und unter Argon destilliert.

Für die Versuche wurden im Autoklaven unter Argonatmosphäre folgende Lösungen des Rhodiums in Form von [(acac)Rh(COD)] (acac=Acetylacetonat-Anion; COD=1,5-Cyclooctadien) (OMG AG & Co. KG, Hanau, DE) als Katalysatorvorstufe in Toluol eingefüllt: Für Versuche mit 100 ppm-m Rhodium 10 ml einer 4,31 millimolaren, für 40 ppm-m die gleiche Menge einer entsprechend verdünnte Lösung. Anschließend wurde die entsprechende Menge der in Toluol gelösten Phosphitverbindung, in der Regel 2 bis 5 Ligandäquivalente pro Rhodium, zugemischt. Durch Zugabe von weiterem Toluol (Die Gesamtmasse an Toluol wurde bestimmt für die GC-Analyse, s.u.) wurde das Anfangsvolumen der Katalysatorlösung auf a) 41,0 ml eingestellt bei beabsichtigter Zugabe von 15 ml des Olefins über die Druckpipette (1-Octen, n-Octenen und Versuche mit erhöhter Konzentration an 2-Penten), oder b) 51,9 ml eingestellt bei beabsichtigter Zugabe von 4,1 ml 2-Penten. Die jeweils eingebrachte Masse an Toluol wurde bestimmt. Einwaagen der Olefine: 1-Octen (10,62 g; 94,64 mmol), n-Octene (10,70 g; 95,35 mmol), 2-Penten 9,75 g; 139,00 mmol. Der Autoklav wurde bei einem Gesamtgasdruck (Synthesegas: Linde; H₂ (99,999%): CO (99,997%) = 1:1) von a) 42 bar für einen Enddruck von 50 bar; b) 12 bar für den Enddruck von 20 bar und c) 7 bar für einen Enddruck von 10 bar unter Rühren (1500 U/min) auf die jeweils angegebenen Temperaturen aufgeheizt. Nach Erreichen der Reaktionstemperatur wurde der Synthesegasdruck auf a) 48,5 bar für einen Enddruck von 50 bar, b) 19,5 bar für einen Enddruck von 20 bar und c) 9,5 bar für einen Enddruck von 10 bar erhöht das jeweils in der Tabelle angegebene Olefin(-gemisch) mit einem in der Druckpipette eingestellten Überdruck von ca. 3 bar zugepresst. Die Reaktion wurde bei konstantem Druck von jeweils 50, 20 bzw. 10 bar (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm, Die quantitative Bestimmung von Restolefin und Aldehyd erfolgte gegen das Lösungsmittel Toluol als internen Standard.

### Ergebnisse der Katalyseversuche

Solvens: Toluol
Ausb. = Ausbeute
p = Druck in [bar]
T = Temperatur in [°C]
t = Zeit in [h]
[Rh] = Rhodiumkonzentration in [ppm]
L/Rh = Verhältnis von Ligand zum Rhodium

Als Vergleichsligand wurde der Ligand **2** verwendet.

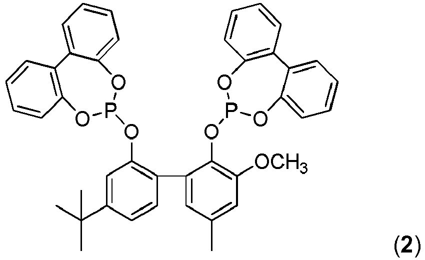

Die erfindungsgemäße Verbindung ist mit * gekennzeichnet.

**Tabelle 1: 1-Octen**

| **Ligand** | **p (bar)** | **T (°C)** | **t (h)** | **[Rh] (ppm)** | **L/Rh** | **Ausb. (%)** |
|---|---|---|---|---|---|---|
| **1*** | 50 | 100 | 4 | 40 | 2 | 91 |
| **2** | 50 | 100 | 4 | 40 | 2 | 90 |

Wie der Tabelle 1 entnommen werden kann, konnte die bereits sehr gute Ausbeute des Liganden **2** nochmals gesteigert werden.

**Tabelle 2: 2-Penten**

| **Ligand** | **p (bar)** | **T (°C)** | **t (h)** | **[Rh] (ppm)** | **L/Rh** | **Ausb. (%)** |
|---|---|---|---|---|---|---|
| **1*** | 20 | 120 | 4 | 100 | 2 | 98 |
| **2** | 20 | 120 | 4 | 100 | 2 | 93 |

Wie der Tabelle 2 entnommen werden kann, konnte die bereits sehr gute Ausbeute des Liganden **2** nochmals merklich gesteigert werden.

Wie die Versuchsergebnisse zeigen, wird die gestellte Aufgabe durch die erfindungsgemäßen Verbindungen gelöst.

Es ist erstmalig gelungen, ein Bisphosphit zu generieren, welches einen unsymmetrischen Biphenol-Flügel-Baustein aufweist und sehr gute Hydroformylierungseigenschaften aufweist. Solche konkreten Strukturen und derartige Liganden waren bis dato gänzlich unbekannt und nicht zugänglich.
Diese Bisphosphite weisen eine neuartige Asymmetrie auf. Das besondere hierbei ist die Asymmetrie innerhalb des Biphenol-Flügel-Bausteins, die zu unsymmetrischen Bisphosphiten führt. Diese unsymmetrischen Bisphosphite sind strukturell somit gänzlich von denen im Stand der Technik beschriebenen Bisphoshiten verschieden, bei denen unsymmetrische Bisphosphit-Liganden durch eine bestimmte Anordnung von symmetrischen Biphenol-Bausteinen generiert werden, indem sich beispielsweise die beiden Flügel-Bausteine unterscheiden, die einzelnen Bausteine (Zentral-Baustein und Flügel-Bausteine) an sich aber symmetrisch sind.

## Patentansprüche

1. Verbindung, welche die allgemeine Struktur I aufweist: 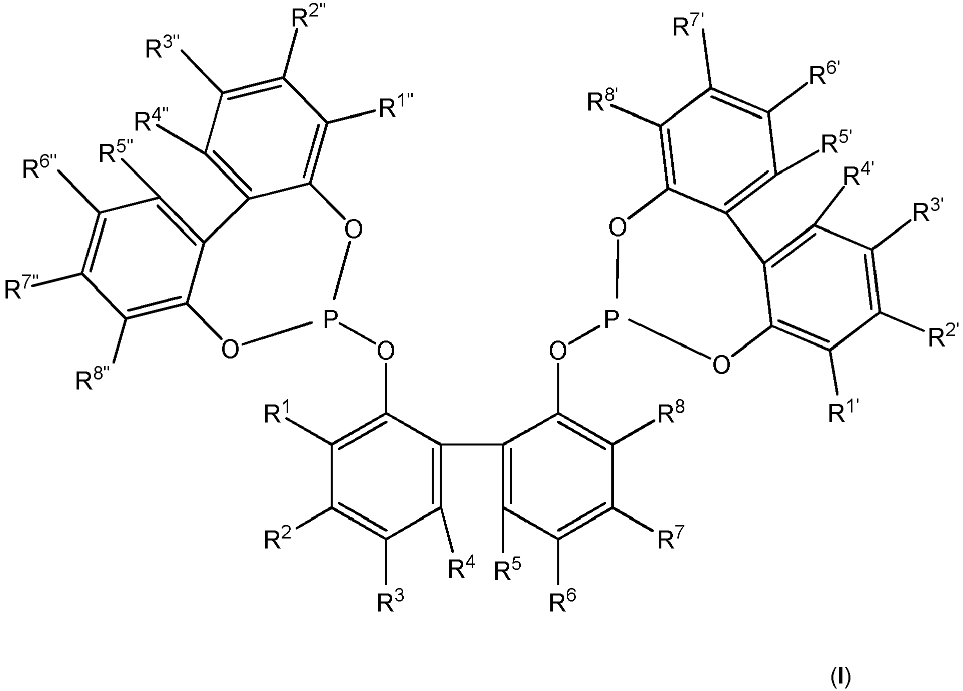 wobei
R¹, R², R³, R⁴, R⁵, R⁶, R⁷, R⁸ ausgewählt sind aus:
- H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, , -CO-(C₁-C₁₂)-Alkyl, -CO-(C₆-C₂₀)-Aryl, -COOH, -OH, -SO₃H -CN, -NH₂, -N[(C₁-C₁₂)-Alkyl]₂;
R¹, R²', R³', ,R⁴', R⁵', R⁶', R⁷', R⁸', R¹", R²", R³", R⁴", R⁵", R⁶", R⁷", R⁸" ausgewählt sind aus:
- H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
wobei die genannten Alkyl- und Arylgruppen wie folgt substituiert sein können:
substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C,₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl,-COOH, -OH, -SO₃H; -SO₃Na, -NO₂, -CN, -NH₂, -N[(C₁-C₁₂)-Alkyl]₂;
und die beiden Reste mindestens eines der vier folgenden Restepaare nicht für den gleichen Rest stehen: R¹' und R⁸', R²' und R⁷', R³' und R⁶', R⁴' und R⁵',
und / oder die beiden Reste mindestens eines der vier folgenden Restepaare nicht für den gleichen Rest stehen: R¹" und R⁸", R²" und R⁷", R³" und R⁶", R⁴" und R⁵".

2. Verbindung nach Anspruch 1,
wobei R¹, R², R³, R⁴, R⁵, R⁶, R⁷, R⁸ ausgewählt sind aus:
- H, -(C₁-C₁₂)-Alkyl, -O-(C₁-C₁₂)-Alkyl, -O-(C₆-C₂₀)-Aryl, -S-Alkyl, -S-Aryl.

3. Verbindung nach einem der Ansprüche 1 oder 2,
wobei R¹', R²', R³', R⁴', R⁵', R⁶', R⁷', R⁸' ausgewählt sind aus:
- H, -(C₁-C₁₂)-Alkyl, -O-(C₁-C₁₂)-Alkyl, -O-(C₆-C₂₀)-Aryl, -S-Alkyl, -S-Aryl.

4. Verbindung nach einem der Ansprüche 1 bis 3,
wobei R¹", R²", R³", R⁴", R⁵", R⁶", R⁷", R⁸" ausgewählt sind aus:
- H, -(C₁-C₁₂)-Alkyl, -O-(C₁-C₁₂)-Alkyl, -O-(C₆-C₂₀)-Aryl, -S-Alkyl, -S-Aryl.

5. Verbindung nach einem der Ansprüche 1 bis 4,
wobei R¹, R², R³, R⁴, R⁵, R⁶, R⁷, R⁸ ausgewählt sind aus:
- H, -(C₁-C₁₂)-Alkyl, -O-(C₁-C₁₂)-Alkyl, -O-(C₆-C₂₀)-Aryl.

6. Verbindung nach einem der Ansprüche 1 bis 5,
wobei R¹', R²', R³', R⁴', R⁵', R⁶', R⁷', R⁸' ausgewählt sind aus:
- H, -(C₁-C₁₂)-Alkyl, -O-(C₁-C₁₂)-Alkyl, -O-(C₆-C₂₀)-Aryl.

7. Verbindung nach einem der Ansprüche 1 bis 6,
wobei R¹", R²", R³", R⁴", R⁵", R⁶", R⁷", R⁸" ausgewählt sind aus:
- H, -(C₁-C₁₂)-Alkyl, -O-(C₁-C₁₂)-Alkyl, -O-(C₆-C₂₀)-Aryl.

8. Verbindung nach einem der Ansprüche 1 bis 7,
wobei R¹, R¹' und R¹" für den gleichen Rest stehen und
R⁸, R⁸' und R⁸" für den gleichen Rest stehen.

9. Verbindung nach einem der Ansprüche 1 bis 8,
wobei R¹' und R⁸' nicht für den gleichen Rest stehen und
R¹" und R⁸" nicht für den gleichen Rest stehen.

10. Verbindung nach einem der Ansprüche 1 bis 9,
wobei die beiden Reste mindestens eines der vier folgenden Restepaare nicht für den gleichen Rest stehen: R¹' und R⁸', R²' und R⁷', R³' und R⁶', R⁴' und R⁵',
und die beiden Reste mindestens eines der vier folgenden Restepaare nicht für den gleichen Rest stehen: R¹" und R⁸", R²" und R⁷", R³" und R⁶", R⁴" und R⁵".

11. Verbindung nach einem der Ansprüche 1 bis 8,
wobei die beiden Reste mindestens eines der vier folgenden Restepaare nicht für den gleichen Rest stehen: R¹' und R⁸', R²' und R⁷', R³, und R⁶', R⁴' und R⁵',
und die beiden Reste der vier folgenden Restepaare für den gleichen Rest stehen: R¹" und R⁸", R²" und R⁷" , R³" und R⁶", R⁴" und R⁵".

12. Verbindung nach einem der Ansprüche 1 bis 10,
welche die Formeln (**1**) aufweist:

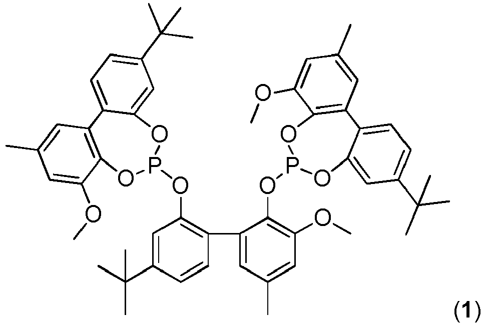

13. Komplex umfassend:
- eine Verbindung nach einem der Ansprüche 1 bis 12,
- ein Metallatom ausgewählt aus: Rh, Ru, Co, Ir.

14. Verwendung einer Verbindung nach einem der Ansprüche 1 bis 12,
zur Katalyse einer Hydroformylierungsreaktion.

15. Verfahren umfassend die Verfahrensschritte:
a) Vorlegen eines Olefins,
b) Zugabe eines Komplexes nach Anspruch 13,
oder einer Verbindung nach einem der Ansprüche 1 bis 12 und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

## Claims

1. Compound having the general structure I: 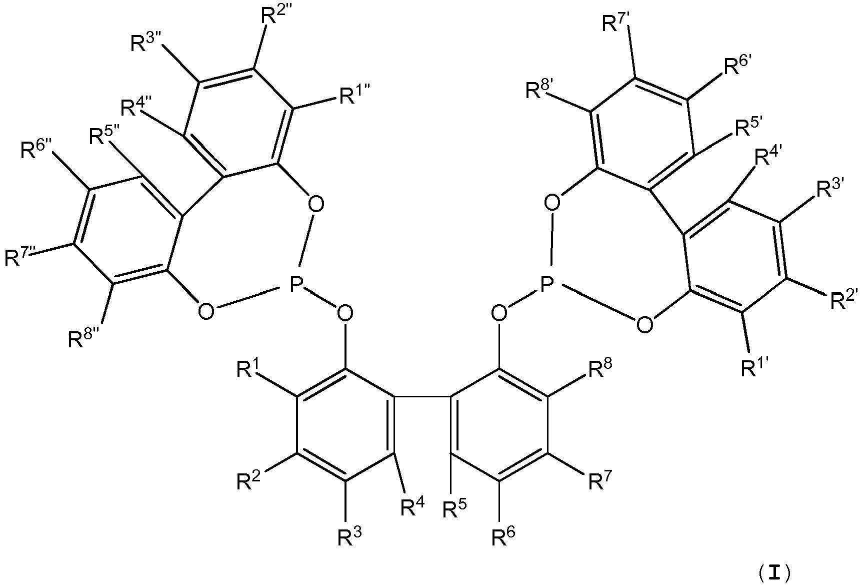 where
R¹, R², R³, R⁴, R⁵, R⁶, R⁷, R⁸ are selected from:
- H, -(C₁-C₁₂)-alkyl, -O-(C₁-C₁₂)-alkyl, -O-(C₆-C₂₀)-aryl, - (C₆-C₂₀)-aryl, -S-alkyl, -S-aryl, halogen, COO-(C₁-C₁₂)-alkyl, CONH-(C₁-C₁₂)-alkyl, -CO-(C₁-C₁₂)-alkyl, -CO-(C₆-C₂₀)-aryl, -COOH, -OH, -SO₃H, -CN, -NH₂, -N[(C₁-C₁₂)-alkyl]₂;
R¹', R²', R³', R⁴', R⁵', R⁶', R⁷', R⁸', R¹", R²", R³", R⁴", R⁵", R⁶", R⁷", R⁸" are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen, COO-(C₁-C₁₂)-alkyl, CONH-(C₁-C₁₂)-alkyl, -CO-(C₁-C₁₂)-alkyl, -CO-(C₆-C₂₀)-aryl, -COOH, -OH, -SO₃H, -NH₂, -N[(C₁-C₁₂)-alkyl]₂;
where the alkyl and aryl groups mentioned may be substituted as follows:
substituted - (C₁-C₁₂) -alkyl groups and substituted -(C₁-C₁₂)-alkoxy groups may have one or more substituents, depending on their chain length; the substituents are each independently selected from -(C₃-C₁₂)-cycloalkyl, -(C₃-C₁₂)-heterocycloalkyl, -(C₆-C₂₀)-aryl, fluorine, chlorine, cyano, formyl, acyl and alkoxycarbonyl;
substituted -(C₆-C₂₀)-aryl groups and -(C₆-C₂₀)-aryl-(C₆-C₂₀)-aryl groups may have one or more substituents, depending on the ring size; these substituents are each independently selected from -H, -(C₁-C₁₂)-alkyl, -O-(C₁-C₁₂)-alkyl, -O-(C₆-C₂₀)-aryl, -(C₆-C₂₀)-aryl, -halogen, -COO-(C₁-C₁₂)-alkyl, -CONH-(C₁-C₁₂)-alkyl, - (C₆-C₂₀)-aryl-CON[(C₁-C₁₂)-alkyl]₂, -CO-(C₁-C₁₂)-alkyl, -CO-(C₆-C₂₀)-aryl, -COOH, -OH, -SO₃H, -SO₃Na, -NO₂, -CN, -NH₂, -N[(C₁-C₁₂)-alkyl]₂;
and the two radicals from at least one of the four following pairs of radicals are not the same radical: R¹' and R⁸', R²' and R⁷', R³' and R⁶', R⁴' and R⁵',
and/or two radicals from at least one of the four following pairs of radicals are not the same radical: R¹" and R⁸", R²" and R⁷", R³" and R⁶", R⁴" and R⁵".

2. Compound according to Claim 1,
where R¹, R², R³, R⁴, R⁵, R⁶, R⁷, R⁸ are selected from:
- H, -(C₁-C₁₂)-alkyl, -O-(C₁-C₁₂)-alkyl, -O-(C₆-C₂₀)-aryl, -S-alkyl, -S-aryl.

3. Compound according to either of Claims 1 and 2,
where R¹, R², R³, R⁴, R⁵, R⁶, R⁷, R⁸ are selected from:
- H, -(C₁-C₁₂)-alkyl, -O-(C₁-C₁₂)-alkyl, -0- (C₆-C₂₀)-aryl.

4. Compound according to any of Claims 1 to 3,
where R¹", R²", R³", R⁴", R⁵", R⁶", R⁷", R⁸" are selected from:
- H, -(C₁-C₁₂)-alkyl, -O-(C₁-C₁₂)-alkyl, -O-(C₆-C₂₀)-aryl.

5. Compound according to any of Claims 1 to 4,
where R¹, R², R³, R⁴, R⁵, R⁶, R⁷, R⁸ are selected from:
- H, -(C₁-C₁₂)-alkyl, -O-(C₁-C₁₂)-alkyl, -O-(C₆-C₂₀)-aryl.

6. Compound according to any of Claims 1 to 5,
where R¹', R²', R³', R⁴', R⁵', R⁶', R⁷', R⁸' are selected from:
- H, - (C₁-C₁₂)-alkyl, -O-(C₁-C₁₂)-alkyl, -O-(C₆-C₂₀)-aryl.

7. Compound according to any of Claims 1 to 6,
where R¹", R²", R³", R⁴", R⁵", R⁶", R⁷", R⁸" are selected from:
- H, -(C₁-C₁₂)-alkyl, -O-(C₁-C₁₂)-alkyl, -O-(C₆-C₂₀)-aryl.

8. Compound according to any of Claims 1 to 7,
where R¹, R¹' and R¹" are the same radical and
R⁸, R⁸' and R⁸" are the same radical.

9. Compound according to any of Claims 1 to 8,
where R¹' and R⁸' are not the same radical and
R¹" and R⁸" are not the same radical.

10. Compound according to any of Claims 1 to 9,
where the two radicals from at least one of the four following pairs of radicals are not the same radical:
R¹' and R⁸', R²' and R⁷', R³' and R⁶', R⁴' and R⁵',
and the two radicals from at least one of the four following pairs of radicals are not the same radical: R¹" and R⁸", R²" and R⁷", R³" and R⁶", R⁴" and R⁵".

11. Compound according to any of Claims 1 to 8,
where the two radicals from at least one of the four following pairs of radicals are not the same radical:
R¹' and R⁸', R²' and R⁷', R³' and R⁶', R⁴' and R⁵',
and the two radicals from at least one of the four following pairs of radicals are the same radical: R¹" and R⁸", R²" and R⁷", R³" and R⁶", R⁴" and R⁵".

12. Compound according to any of Claims 1 to 10, having the formulae (**1**) :

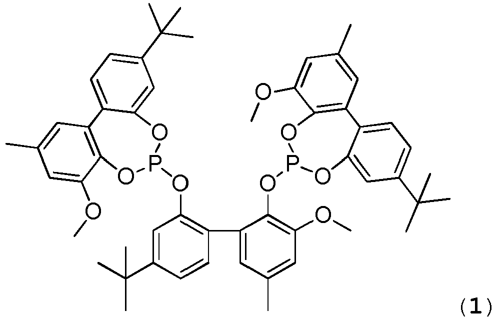

13. Complex comprising:
- a compound according to any of Claims 1 to 12,
- a metal atom selected from: Rh, Ru, Co, Ir.

14. Use of a compound according to any of Claims 1 to 12
for catalysing a hydroformylation reaction.

15. Process comprising the following process steps:
a) initially charging an olefin,
b) adding a complex according to Claim 13,
or a compound according to any of Claims 1 to 12 and a substance having a metal atom selected from: Rh, Ru, Co, Ir,
c) feeding in H₂ and CO,
d) heating the reaction mixture, with conversion of the olefin to an aldehyde.

## Revendications

1. Composé, qui présente la structure générale **I :** 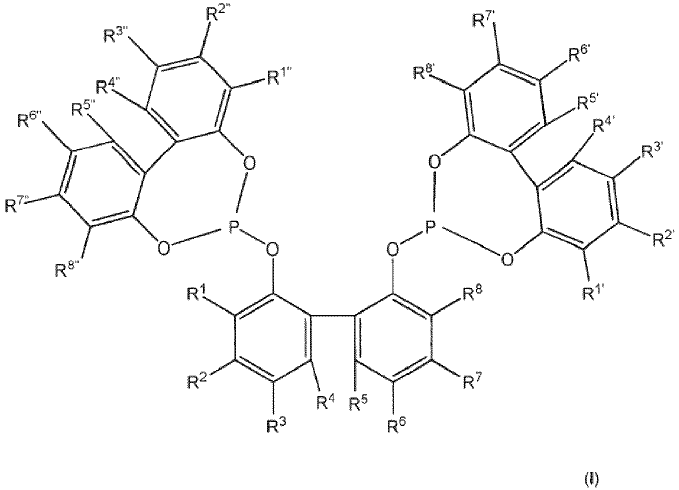 dans laquelle
R¹, R², R³, R⁴, R⁵, R⁶, R⁷, R⁸ sont choisis parmi :
- H, -alkyle(C₁-C₁₂), -O-alkyle(C₁-C₁₂), -O-aryle(C₆-C₂₀), -aryle(C₆-C₂₀), -S-alkyle, -S-aryle, des atomes d'halogène, COO-alkyle(C₁-C₁₂), CONH-alkyle(C₁-C₁₂), -CO-alkyle(C₁-C₁₂), -CO-aryle (C₆-C₂₀), -COOH, -OH, -SO₃H, -CN, -NH₂, -N[alkyle(C₁-C₁₂)]₂ ;
R¹', R²', R³', R⁴', R⁵', R⁶', R⁷', R⁸', R¹", R²", R³", R⁴", R⁵", R⁶", R⁷", R⁸" sont choisis parmi .
- H, -alkyle(C₁-C₁₂), -O-alkyle(C₁-C₁₂), -O-aryle(C₆-C₂₀), -aryle(C₆-C₂₀), -S-alkyle, -S-aryle, des atomes d'halogène, COO-alkyle(C₁-C₁₂), CONH-alkyle(C₁-C₁₂), -CO-alkyle(C₁-C₁₂), -CO-aryle(C₆-C₂₀), -COOH, -OH, -SO₃H, - NH₂, -N[alkyle(C₁-C₁₂)]₂;
les groupes alkyle et aryle nommés pouvant être substitués comme suit :
les groupes -alkyle(C₁-C₁₂) substitués et les groupes -alcoxy(C₁-C₁₂) substitués peuvent comporter, en fonction de leur longueur de chaîne, un ou plusieurs substituants ; les substituants sont choisis, indépendamment les uns des autres, parmi -cycloalkyle(C₃-C₁₂), -hétérocycloalkyle(C₃-C₁₂), -aryle(C₆-C₂₀), fluoro, chloro, cyano, formyle, acyle ou alcoxycarbonyle ;
les groupes -aryle(C₆-C₂₀) substitués et les groupes -aryl(C₆-C₂₀)-aryle(C₆-C₂₀) substitués peuvent comporter, en fonction de la taille du cycle, un ou plusieurs substituants ; ces substituants sont choisis, indépendamment les uns des autres, parmi -H, -alkyle(C₁-C₁₂), -O-alkyle(C₁-C₁₂), -O-aryle(C₆-C₂₀), -aryle(C₆-C₂₀), halogéno, -COO-alkyle(C₁-C₁₂), -CONH-alkyle(C₁-C₁₂), -aryl(C₆-C₂₀)-CON[alkyle(C₁-C₁₂)]₂, -CO-alkyle(C₁-C₁₂), -CO-aryle(C₆-C₂₀), -COOH, -OH, -SO₃H ; -SO₃Na, -NO₂, -CN, -NH₂, -N[alkyle(C₁-C₁₂)]₂ ;
et les deux radicaux d'au moins l'une des quatre paires de radicaux suivantes ne représentent pas le même radical : R^{1'} et R^{8'}, R^{2'} et R^{7'}, R^{3'} et R^{6'}, R^{4'} et R^{5'}, et/ou les deux radicaux d'au moins l'une des quatre paires de radicaux suivantes ne représentent pas le même radical : R^{1"} et R^{8''}, R^{2"} et R^{7"}, R^{3"} et R^{6"}, R^{4"} et R^{5"}.

2. Composé selon la revendication 1,
dans lequel R¹, R², R³, R⁴, R⁵, R⁶, R⁷, R⁸ sont choisis parmi :
- H, -alkyle(C₁-C₁₂), -O-alkyle(C₁-C₁₂), -O-aryle(C₆-C₂₀), -S-alkyle, -S-aryle.

3. Composé selon l'une quelconque des revendications 1 et 2,
dans lequel R^{1'}, R^{2'}, R^{3'}, R^{4'}, R^{5'}, R^{6'}, R^{7'}, R^{8'} sont choisis parmi :
- H, -alkyle(C₁-C₁₂), -O-alkyle(C₁-C₁₂), -O-aryle(C₆-C₂₀), -S-alkyle, -S-aryle.

4. Composé selon l'une quelconque des revendications 1 à 3,
dans lequel R^{1"}, R^{2"}, R^{3"}, R^{4"}, R^{5"}, R^{6"}, R^{7"}, R^{8"} sont choisis parmi :
- H, -alkyle(C₁-C₁₂), -O-alkyle(C₁-C₁₂), -O-aryle(C₆-C₂₀), -S-alkyle, -S-aryle.

5. Composé selon l'une quelconque des revendications 1 à 4,
dans lequel R¹, R², R³, R⁴, R⁵, R⁶, R⁷, R⁸ sont choisis parmi :
- H, -alkyle(C₁-C₁₂), -O-alkyle(C₁-C₁₂), -O-aryle(C₆-C₂₀).

6. Composé selon l'une quelconque des revendications 1 à 5,
dans lequel R^{1'}, R^{2'}, R^{3'}, R^{4'}, R^{5'}, R^{6'}, R^{7'}, R^{8'} sont choisis parmi :
- H, -alkyle(C₁-C₁₂), -O-alkyle(C₁-C₁₂), -O-aryle(C₆-C₂₀).

7. Composé selon l'une quelconque des revendications 1 à 6,
dans lequel R^{1"}, R^{2"}, R^{3"}, R^{4"}, R^{5"}, R^{6"}, R^{7"}, R^{8"} sont choisis parmi :
- H, -alkyle(C₁-C₁₂), -O-alkyle(C₁-C₁₂), -O-aryle(C₆-C₂₀).

8. Composé selon l'une quelconque des revendications 1 à 7,
dans lequel R¹, R^{1'} et R^{1"} représentent le même radical et
R⁸, R^{8'} et R^{8"} représentent le même radical.

9. Composé selon l'une quelconque des revendications 1 à 8,
dans lequel R^{1'} et R^{8'} ne représentent pas le même radical et
R^{1"} et R^{8"} ne représentent pas le même radical.

10. Composé selon l'une quelconque des revendications 1 à 9,
dans lequel les deux radicaux d'au moins l'une des quatre paires de radicaux suivantes ne représentent pas le même radical : R^{1'} et R^{B'}, R^{2'} et R^{7'}, R^{3'} et R^{6'}, R^{4'} et R^{5'},
et les deux radicaux d'au moins l'une des quatre paires de radicaux suivantes ne représentent pas le même radical : R^{1"} et R^{8''}, R^{2"} et R^{7"}, R^{3"} et R^{6"}, R^{4"} et R^{5"}.

11. Composé selon l'une quelconque des revendications 1 à 8,
dans lequel les deux radicaux d'au moins l'une des quatre paires de radicaux suivantes ne représentent pas le même radical : R^{1'} et R^{8'}, R^{2'} et R^{7'}, R^{3'} et R^{6'}, R^{4'} et R^{5'},
et les deux radicaux des quatre paires de radicaux suivantes représentent le même radical : R^{1"} et R^{8''}, R^{2"} et R^{7"} R^{3"} et R^{6"}, R^{4"} et R^{5"}.

12. Composé selon l'une quelconque des revendications 1 à 10,
qui présente les formules (**1**) :

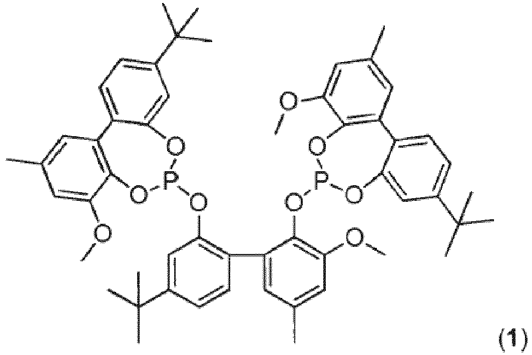

13. Complexe comprenant
- un composé selon l'une quelconque des revendications 1 à 12,
- un atome métallique choisi parmi : Rh, Ru, Co, Ir.

14. Utilisation d'un composé selon l'une quelconque des revendications 1 à 12,
pour la catalyse d'une réaction d'hydroformylation.

15. Procédé comprenant les étapes de processus :
a) disposition au préalable d'une oléfine,
b) addition d'un complexe selon la revendication 13,
ou d'un composé selon l'une quelconque des revendications 1 à 12 et d'une substance qui comporte un atome métallique choisi parmi : Rh, Ru, Co, Ir,
c) introduction de H₂ et CO,
d) chauffage du mélange réactionnel, de sorte que l'oléfine est convertie en un aldéhyde.
